(19) Europäisches Patentamt / European Patent Office / Office européen des brevets

(11) **EP 3 891 502 B1**

(12) **EUROPÄISCHE PATENTSCHRIFT**

(45) Veröffentlichungstag und Bekanntmachung des Hinweises auf die Patenterteilung:
**19.10.2022 Patentblatt 2022/42**

(21) Anmeldenummer: **19817642.2**

(22) Anmeldetag: **05.12.2019**

(51) Internationale Patentklassifikation (IPC):
**G01N 33/28** [(2006.01)]

(52) Gemeinsame Patentklassifikation (CPC):
**G01N 33/2841**

(86) Internationale Anmeldenummer:
**PCT/EP2019/083870**

(87) Internationale Veröffentlichungsnummer:
**WO 2020/115232 (11.06.2020 Gazette 2020/24)**

(54) **ÜBERWACHUNG VON ENTSTEHENDEN GASEN IN EINEM ISOLIERMITTELHAUSHALT**

MONITORING OF GASES PRODUCED IN AN INSULATING-MEANS HOUSEHOLD

SURVEILLANCE DES GAZ PRODUITS DANS UN FOYER COMPRENANT DES PRODUITS ISOLANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.12.2018 DE 102018131388**

(43) Veröffentlichungstag der Anmeldung:
**13.10.2021 Patentblatt 2021/41**

(73) Patentinhaber: **Maschinenfabrik Reinhausen GmbH**
**93059 Regensburg (DE)**

(72) Erfinder:
• **FROTSCHER, Rainer**
**93128 Regenstauf (DE)**
• **SACHSENHAUSER, Andreas**
**84066 Mallersdorf (DE)**

(56) Entgegenhaltungen:
EP-A1- 3 073 248        WO-A1-2007/115807
US-A1- 2014 165 704

• LIN JUN ET AL: "Online Monitoring Data Cleaning of Transformer Considering Time Series Correlation", 2018 IEEE/PES TRANSMISSION AND DISTRIBUTION CONFERENCE AND EXPOSITION (T&D), IEEE, 16. April 2018 (2018-04-16), Seiten 1-9, XP033391026, DOI: 10.1109/TDC.2018.8440521 [gefunden am 2018-08-17]
• NAN YAO ET AL: "Transformer fault detection based on infrared power image", ACTA TECHNICA, Bd. 62, Nr. 2A, 2017, Seiten 237-244, XP055675452,

**Beschreibung**

Die Erfindung betrifft ein Verfahren zur Überwachung von entstehenden Gasen in einem Isoliermittelhaushalt, der mit einem Überschaltwiderstand eines Laststufenschalters in Kontakt steht sowie einen entsprechenden Laststufenschalter.

Die Entstehung bestimmter gelöster Gase in einem Isoliermittel, zum Beispiel einem Isolieröl, in dem sich ein Laststufenschalter befindet, wie beispielsweise bei einem Transformator oder einem anderen elektrischen Betriebsmittel aus dem Gebiet der Hochspannungstechnik, kann als Indikator für Fehlfunktionen oder bevorstehende Fehlfunktionen des elektrischen Betriebsmittels oder des Laststufenschalters dienen. Aus dem Stand der Technik sind Verfahren bekannt, die sich mit der Zustandsbewertung und der Analyse von gelösten Gasen im Isoliermittel von Hochspannungsgeräten befassen. Die Gase können dabei durch den Laststufenschalter und/oder sonstige Komponenten des Betriebsmittels erzeugt werden.

Laststufenschalter werden zur unterbrechungslosen Umschaltung zwischen Wicklungsanzapfungen eines Transformators eingesetzt. Bekannte Laststufenschalter bestehen üblicherweise aus einem Wähler zur leistungslosen Anwahl der jeweiligen Wicklungsanzapfung des Transformators, auf die umgeschaltet werden soll, und einem Lastumschalter zur eigentlichen Umschaltung von der bisherigen auf die neue, vorgewählte Wicklungsanzapfung. Üblicherweise wird der Wähler im Isoliermittel des Transformators und der Lastumschalter in einem separaten Isolierölgefäß im Transformatorgehäuse untergebracht. Ebenfalls aus dem Stand der Technik bekannt ist es jedoch, den gesamten Laststufenschalter ohne separates Gefäß für den Lastumschalter im Transformatorgehäuse zu befestigen, sodass sich Lastumschalter und Transformator in demselben Isoliermittelhaushalt befinden.

Bei bekannten Lastumschaltern nach dem Widerstandsschnellschaltprinzip werden Überschaltwiderstände zur Begrenzung des Laststroms während der zwischenzeitlich gleichzeitigen Kontaktierung der aktuell beschalteten und der vorgewählten, neuen Wicklungsanzapfung eingesetzt. Während dieses Vorgangs der Lastumschaltung werden die Überschaltwiderstände erwärmt und es entstehen sogenannte Wärmegase, die auch bei ordnungsgemäßer Funktion des Laststufenschalters zu erwarten sind.

LIN JUN ET AL: "Online Monitoring Data Cleaning of Transformer Considering Time Series Correlation",2018 IEEE/PES TRANSMISSION AND DISTRIBUTION CONFERENCE AND EXPOSITION (T&D), IEEE, 16. April 2018 (2018-04-16), Seiten 1-9, NAN YAO ET AL: "Transformer fault detection based on infrared power image",ACTA TECHNICA, Bd. 62, Nr. 2A, 2017, Seiten 237-244 sowie EP 3 073 248 offenbaren verwandte Verfahren zur Überwachung in einem Isoliermittelhaushalt.

Bekannte Verfahren beziehungsweise Vorrichtungen zur Überwachung von entstehenden Gasen in einem Isoliermittelhaushalt und bekannte Verfahren zur Zustandsbewertung von mit Isoliermittel befüllten, elektrischen Betriebsmitteln mit Laststufenschaltern berücksichtigen beispielsweise nicht die bei der Beanspruchung der Überschaltwiderstände entstehenden Wärmegase und können daher zu einer falschen Interpretation der Gasanalyse führen und die Zustandsbewertung des elektrischen Betriebsmittels beeinträchtigen. Eine separate oder zusätzliche Analyse der durch den Laststufenschalter entstehenden Gase ist aufwändig und kostspielig. Im Falle eines gemeinsamen Isoliermittelhaushalts ist eine Trennung der Gasanteile nach verursachenden Komponenten des Betriebsmittels ohnehin nicht möglich, so dass eine solche separate Analyse nicht durchführbar ist. Daher ist es Aufgabe der Erfindung, ein verbessertes Konzept zur Überwachung von entstehenden Gasen in einem Isoliermittelhaushalt mit Laststufenschalter anzugeben, das eine vereinfachte Berücksichtigung von durch den Laststufenschalter erzeugten Gasen ermöglicht.

Diese Aufgabe wird durch die Gegenstände der unabhängigen Ansprüche gelöst. Weitere Ausführungsformen sind in den abhängigen Ansprüchen beschrieben.

Gemäß einem ersten Aspekt des verbesserten Konzepts wird ein Verfahren zur Überwachung von entstehenden Gasen in einem Isoliermittelhaushalt angegeben, wobei der Isoliermittelhaushalt mit einem Überschaltwiderstand eines Laststufenschalters in Kontakt steht. Dabei wird ein zeitlicher Verlauf einer Widerstandstemperatur des Überschaltwiderstandes während eines Belastungszeitraums ermittelt und aus dem zeitlichen Verlauf der Widerstandstemperatur wenigstens ein Kennwert zur Charakterisierung der entstehenden Gase bestimmt.

Die Ermittlung der Widerstandstemperatur und der daraus ableitbare Kennwert zur Charakterisierung der entstehenden Gase erlauben es, die Gase, welche im Isoliermittel des Laststufenstufenschalters, eines Transformators oder eines anderen elektrischen Betriebsmittels mit dem Laststufenschalter durch die Erwärmung der Überschaltwiderstände des Laststufenschalters entstehen, zu überwachen und so eine genauere Aussage über den Zustand des Isoliermittels zu treffen, ohne auf eine dedizierte Gasanalyse der durch den Laststufenschalter erzeugten Gase zurückgreifen zu müssen.

Gemäß zumindest einer Ausführungsform ist der Laststufenschalter nach dem Widerstandsschnellschaltprinzip ausgebildet.

Gemäß zumindest einer Ausführungsform handelt es sich bei dem Isoliermittel um eine Isolierflüssigkeit, insbesondere Isolieröl, beispielsweise ein mineralisches Transformatorenöl oder eine alternative Isolierflüssigkeit, wie zum Beispiel einen synthetischen Ester.

**EP 3 891 502 B1**

Gemäß zumindest einer Ausführungsform beinhalten die entstehenden Gase Wasserstoff, Methan, Ethan, Ethen, Propan oder Propen.

Gemäß zumindest einer Ausführungsform gilt als Belastungszeitraum des Überschaltwiderstandes insbesondere ein Zeitraum, in dem ein Laststrom über den Überschaltwiderstand fließt und diesen erwärmt.

Gemäß zumindest einer Ausführungsform wird der wenigstens eine Kennwert anhand einer Kurve des zeitlichen Verlaufs der Widerstandstemperatur bestimmt.

Die Kurve stellt insbesondere die Funktion der Widerstandstemperatur über die Zeit dar.

Gemäß zumindest einer Ausführungsform umfasst der Kennwert eine Gasmenge.

Gemäß zumindest einer Ausführungsform des Verfahrens wird die Gasmenge durch Bestimmen eines Flächeninhalts ermittelt, der von der Kurve des zeitlichen Verlaufs der Widerstandstemperatur eingeschlossen wird.

Der Flächeninhalt kann insbesondere die gesamte Fläche, die von der Kurve eingeschlossen wird, oder nur ein Teil dieser Fläche sein.

Gemäß zumindest einer Ausführungsform liegt der Flächeninhalt oberhalb eines vorbestimmten Temperaturmindestwerts.

Der Flächeninhalt kann beispielsweise durch Integration oder näherungsweise Integration der Widerstandstemperatur über die Zeit bestimmt werden.

Der Temperaturmindestwert ist insbesondere kennzeichnend für eine Temperatur, ab der die genannten Wärmegase während dem Betrieb des Laststufenschalters in signifikantem, insbesondere messbarem, Umfang entstehen. Dieser Wert ist beispielsweise abhängig vom verwendeten Isoliermittel. Für ein mineralisches Transformatorenöl beträgt dieser Wert beispielsweise in etwa 100°C. Die Ermittlung des Temperaturmindestwerts erfolgt beispielsweise vorab durch Versuche.

Gemäß zumindest einer Ausführungsform wird die Gasmenge mit einem festgelegten Grenzwert verglichen und eine Meldung ausgegeben, wenn die Gasmenge gleich dem festgelegten Grenzwert ist oder den festgelegten Grenzwert überschreitet.

Insbesondere ist der Grenzwert derart festgelegt, dass die bei ordnungsgemäßer Funktion des Laststufenschalters zu erwartende Gasmenge unter diesem Grenzwert liegt. Dies ist insbesondere vorteilhaft, wenn sich Laststufenschalter und Transformator in einem gemeinsamen Isoliermittelhaushalt befinden. Beispielsweise wird dann das Ergebnis einer Gasanalyse des Transformatoröls nicht dahingehend beeinträchtigt, dass aufgrund der im Transformatoröl vorhandenen Gasmenge auf einen Fehler geschlossen wird, obwohl die vorhandene Gasmenge gar nicht auf einen Fehlerfall des Transformators, sondern lediglich auf die bei der Lastumschaltung entstandenen Wärmegase zurückzuführen ist.

Gemäß zumindest einer Ausführungsform umfasst der Kennwert eine Gaszusammensetzung.

Gemäß zumindest einer Ausführungsform wird die Gaszusammensetzung ermittelt durch die Kurve des zeitlichen Verlaufs der Widerstandstemperatur und einer maximalen Widerstandstemperatur.

Insbesondere stellt die Kurve des zeitlichen Verlaufs der Widerstandstemperatur das Abkühlverhalten des Überschaltwiderstands nach der Belastung durch eine Lastumschaltung dar.

Beispielsweise kühlt ein Überschaltwiderstand je nach Auslegung und konkretem Anwendungsfall nach einer Lastumschaltung innerhalb von 10 - 15 Sekunden von der maximalen Widerstandstemperatur auf eine Isoliermitteltemperatur ab.

Beispielsweise kann die aktuelle, maximale Widerstandstemperatur in gewissen Zeitabschnitten ermittelt werden und die bei den jeweiligen Temperaturen vorhandene Gaszusammensetzung, also welche Art und Menge von gelösten Gasen im Isoliermittel vorhanden sind, durch die Entnahme von Isoliermittelproben ermittelt werden.

Gemäß zumindest einer Ausführungsform kann durch diese Versuche wenigstens eine Lookup-Tabelle erstellt werden, die beinhaltet, bei welcher Widerstandstemperatur welche Gase entstehen und somit welche Gaszusammensetzung im Isoliermittel vorhanden ist.

Gemäß zumindest einer Ausführungsform stellt die Kurve des zeitlichen Verlaufs der Widerstandstemperatur eine Exponentialfunktion oder näherungsweise eine Exponentialfunktion dar. Insbesondere kann der zeitliche Verlauf der Widerstandstemperatur beginnend von der maximalen Widerstandstemperatur exponentiell oder näherungsweise exponentiell abfallen.

Gemäß zumindest einer Ausführungsform umfasst die Ermittlung der Widerstandstemperatur eine Messung des Laststromes.

Der Laststrom des Transformators wird beispielsweise über einen Stromwandler gemessen.

Gemäß zumindest einer Ausführungsform kann aus dem gemessenen Laststrom $I_L$, der Temperatur des Isoliermittels $T_{IM}$ und applikationsspezifischen Auslegungsparametern des Laststufenschalters die maximale Widerstandstemperatur beispielsweise wie folgt berechnet oder angenähert werden:

3

$$T_{max} = T_{IM} + \left( I_L{}^2 \times t_1 + \left( \frac{U_{St}}{R_{\ddot{u}}} \right)^2 \times t_2 \right) \times \frac{0{,}271}{F^2}$$

mit $F = AnzPar \times \pi \times \frac{d^2}{4}$ als effektive Querschnittsfläche eines Überschaltwiderstandes [mm$^2$].

Die applikationsspezifischen Auslegungsparameter des Laststufenschalters, welche in dieser Formel benötigt werden, sind die Stufenspannung $U_{St}$, die Größe des Überschaltwiderstandes $R_{\ddot{u}}$, die Widerstandsbelastungszeiten in jeweiligen Schaltschritten $t_1$ und $t_2$, die Anzahl der parallelen Drähte je Widerstand *AnzPar* und der Drahtdurchmesser *d*.

Gemäß zumindest einer Ausführungsform umfasst die Ermittlung der Widerstandstemperatur eine Messung der Widerstandstemperatur.

Die Messung der Widerstandstemperatur kann direkt und kontinuierlich während dem Belastungszeitraum oder während einem Teil des Belastungszeitraums durch beispielsweise einen Temperatursensor oder ein Thermoelement am Überschaltwiderstand erfolgen.

Gemäß einem zweiten Aspekt des verbesserten Konzepts wird außerdem ein Verfahren zur Zustandsbewertung eines mit Isoliermittel befüllten, elektrischen Betriebsmittels angegeben, wobei das Betriebsmittel einen Laststufenschalter umfasst und das Betriebsmittel und der Laststufenschalter einen gemeinsamen Isoliermittelhaushalt aufweisen, und wobei das Verfahren zur Zustandsbewertung ein Verfahren zur Überwachung von entstehenden Gasen gemäß dem ersten Aspekt des verbesserten Konzepts umfasst.

Gemäß zumindest einer Ausführungsform ist das elektrische Betriebsmittel als Transformator ausgebildet.

Gemäß zumindest einer Ausführungsform umfasst das Verfahren zur Zustandsbewertung eine Analyse, insbesondere eine Gasanalyse, beispielsweise eine Analyse der Zusammensetzung und/oder wenigstens einer Konzentration gelöster Gase, des Isoliermittels, wobei ein Ergebnis der Analyse abhängig von dem wenigstens einen Kennwert angepasst wird.

Beispielsweise wird das Ergebnis einer Gasanalyse des Transformatorenöls derart angepasst, dass die bei der Betätigung des Laststufenschalters entstandenen Wärmegase bei der Zustandsbewertung des Isoliermittels berücksichtigt, beispielsweise herausgerechnet werden.

Gemäß dem verbesserten Konzept wird außerdem ein Laststufenschalter mit Überschaltwiderstand angegeben, wobei der Laststufenschalter derart ausgelegt ist, dass der Überschaltwiderstand während dem Betrieb des Laststufenschalters mit einem Isoliermittelhaushalt in Kontakt steht. Dabei weist der Laststufenschalter eine Auswerteeinrichtung zur Überwachung entstehender Gase in dem Isoliermittelhaushalt auf, die dazu eingerichtet ist, einen Verlauf einer Widerstandstemperatur des Überschaltwiderstandes während eines Belastungszeitraums zu ermitteln und aus dem zeitlichen Verlauf der Widerstandstemperatur wenigstens einen Kennwert zur Charakterisierung der entstehenden Gase zu bestimmen.

Gemäß zumindest einer Ausführungsform ist der Laststufenschalter derart ausgelegt, dass er in einem elektrischen Betriebsmittel verbaut werden kann, wobei es sich bei dem Isoliermittelhaushalt um einen gemeinsamen Isoliermittelhaushalt von Betriebsmittel und Laststufenschalter handelt.

Gemäß zumindest einer Ausführungsform weist der Laststufenschalter kein eigenes Gehäuse auf, sondern ist in einem Gehäuse, beispielsweise Tank oder Kessel, des elektrischen Betriebsmittels angeordnet.

Gemäß zumindest einer Ausführungsform weist der Stufenschalter ein offenes Gehäuse auf und ist mit dem offenen Gehäuse in dem Gehäuse des elektrischen Betriebsmittels angeordnet.

Gemäß zumindest einer Ausführungsform umfasst der Laststufenschalter einen Temperatursensor zur Messung der Widerstandstemperatur.

Weitere Ausgestaltungsformen des Laststufenschalters gemäß dem verbesserten Konzept ergeben sich unmittelbar aus den verschiedenen Ausgestaltungsformen der Verfahren gemäß dem ersten und zweiten Aspekt und umgekehrt.

Im Folgenden wird die Erfindung anhand beispielhafter Ausführungsformen unter Bezug auf die Zeichnungen im Detail erklärt. Komponenten, die funktionell identisch sind oder einen identischen Effekt haben, können mit identischen Bezugszeichen versehen sein. Identische Komponenten oder Komponenten mit identischer Funktion sind unter Umständen nur bezüglich der Figur erklärt, in der sie zuerst erscheinen. Die Erklärung wird nicht notwendigerweise in den darauffolgenden Figuren wiederholt.

Die Zeichnungen zeigen in

FIG. 1 einen schematischen Aufbau eines elektrischen Betriebsmittels mit einer beispielhaften Ausführungsform eines Laststufenschalters nach dem verbesserten Konzept;

FIG. 2 einen schematischen Aufbau eines weiteren Betriebsmittels mit einer weiteren beispielhaften Ausführungsform eines Laststufenschalters nach dem verbesserten Konzept; und

FIG. 3    eine beispielhafte Darstellung einer Kurve des zeitlichen Verlaufs einer Widerstandstemperatur.

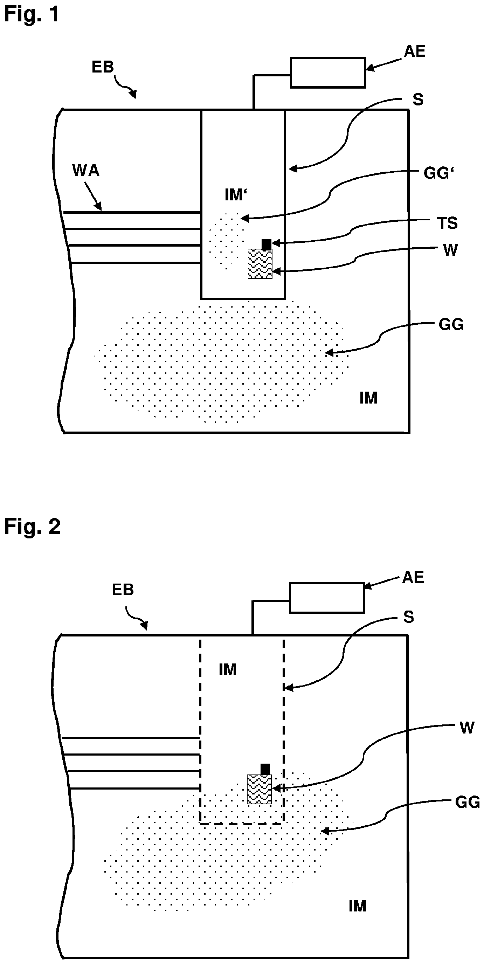

FIG. 1 zeigt ein elektrisches Betriebsmittel EB, beispielsweise einen Transformator, mit einem Gehäuse oder Tank, welches zumindest teilweise mit einem Isoliermittel IM, beispielsweise Transformatorenöl, gefüllt ist. Des Weiteren ist in dem Gehäuse des elektrischen Betriebsmittels ein Laststufenschalter S angeordnet, welcher ebenfalls ein Gehäuse, das zumindest teilweise mit Isoliermittel IM' gefüllt ist, aufweist und mit Wicklungsanzapfungen WA des Transformators verbunden ist. In dem Gehäuse des Laststufenschalters ist ein Überschaltwiderstand W angeordnet. Der Laststufenschalter kann jedoch auch mehrere Widerstände aufweisen. Sowohl in dem Isoliermittel in dem Transformatorgehäuse als auch in dem Isoliermittel in dem Laststufenschaltergehäuse können verschiedene Gase GG, GG' gelöst vorliegen, die zumindest teilweise durch die Erwärmung der Widerstände W entstehen. Zur Überwachung der Entstehung dieser Wärmegase weist der Laststufenschalter eine Auswerteeinrichtung AE auf.

Optional umfasst der Laststufenschalter S einen Temperatursensor TS zur Messung der Widerstandstemperatur. Der Temperatursensor TS ist beispielsweise in solchen Ausführungsformen nicht erforderlich, bei denen die Widerstandstemperatur basierend auf einer Messung des Laststromes bestimmt wird.

In FIG. 2 ist eine weitere, beispielhafte Ausführungsform des verbesserten Konzepts schematisch dargestellt, bei welcher das elektrische Betriebsmittel EB und der Laststufenschalter S einen gemeinsamen Isoliermittelhaushalt IM aufweisen, mit dem der Überschaltwiderstand W des Laststufenschalters S in Kontakt steht. Der Laststufenschalter S kann beispielsweise ein offenes Gehäuse oder gar kein Gehäuse aufweisen, was durch die gestrichelte Linie dargestellt ist. Auch hier können verschiedene gelöste Gase GG im gemeinsamen Isoliermittelhaushalt IM vorhanden sein, die zumindest teilweise durch die Erwärmung des Überschaltwiderstands W entstehen.

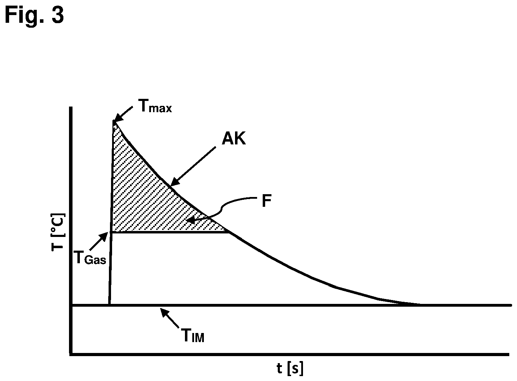

FIG. 3 zeigt eine beispielhafte Darstellung der Widerstandstemperatur T über die Zeit t bei einem Schaltvorgang des Laststufenschalters. Die Abbildung beinhaltet eine Kurve AK, die das Abkühlverhalten eines Überschaltwiderstandes bei einer Lastumschaltung darstellt. Anhand der Kurve ist erkennbar, dass die Temperatur zunächst konstant und gleich der Temperatur des Isoliermittels $T_{IM}$ ist. Dann erfolgt im Zuge der Umleitung des Laststroms über den Widerstand in kurzer Zeit eine starke Erwärmung des Widerstands auf eine maximale Widerstandstemperatur $T_{max}$, gefolgt von einem langsameren, stetigen Abfall der Temperatur bis zurück zur Isoliermitteltemperatur $T_{IM}$. Die schraffierte Fläche F bildet den Bereich unterhalb der Kurve ab, der für die Gasentstehung im Isoliermittel maßgeblich ist. Dieser Bereich liegt oberhalb einer bestimmten Temperatur $T_{Gas}$, beispielsweise 100°C, ab der in signifikanter Menge Wärmegase entstehen und die über Versuche ermittelt werden kann. Folglich kann über die Abkühlkurve zum einen das Zeitfenster ermittelt werden, in dem der Widerstand nach einer Lastumschaltung wieder vollständig bis auf die Isoliermitteltemperatur abkühlt und zum anderen das Zeitfenster, in welchem er sich in einem Temperaturbereich befindet, in dem Wärmegase entstehen.

Über die Größe des schraffierten Flächeninhalts kann die entstehende Gasmenge ermittelt werden. Das heißt beispielsweise, je größer die Fläche oberhalb $T_{Gas}$, desto größer die im Isoliermittel entstandene Gasmenge. Anhand der jeweiligen Höchsttemperatur zum jeweiligen Zeitpunkt kann zudem eine Aussage über die Gaszusammensetzung getroffen werden, das heißt welche Art von Gasen im Isoliermittel entstehen.

Bei kontinuierlicher Temperaturmessung am Überschaltwiderstand, beispielweise mit einem Temperatursensor, kann durch Versuche pro Zeitabschnitt eine typische Gaszusammensetzung ermittelt werden. Die Ergebnisse können beispielsweise in einer Lookup-Tabelle gesammelt werden und bei künftigen Analysen als Interpretationshilfe dienen.

## BEZUGSZEICHEN

| | |
|---|---|
| EB | elektrisches Betriebsmittel |
| S | Laststufenschalter |
| WA | Wicklungsanzapfungen |
| AE | Auswerteeinrichtung |
| W | Überschaltwiderstand |
| IM, IM' | Isoliermittel(-haushalt) |
| GG, GG' | gelöstes Gas |
| TS | Temperatursensor |
| AK | (Abkühlungs-) Kurve |
| $T_{max}$ | maximale Widerstandstemperatur |
| $T_{Gas}$ | für Gasentstehung maßgebliche Mindesttemperatur |
| $T_{IM}$ | Isoliermitteltemperatur |

F       Flächeninhalt

## Patentansprüche

1. Verfahren zur Überwachung von entstehenden Gasen (GG, GG') in einem Isoliermittelhaushalt (IM, IM'), wobei der Isoliermittelhaushalt (IM, IM') mit einem Überschaltwiderstand (W) eines Laststufenschalters (S) in Kontakt steht, und wobei

   - ein zeitlicher Verlauf einer Widerstandstemperatur des Überschaltwiderstandes (W) während eines Belastungszeitraums ermittelt wird,
   - aus dem zeitlichen Verlauf der Widerstandstemperatur wenigstens ein Kennwert zur Charakterisierung der entstehenden Gase (GG, GG') bestimmt wird.

2. Verfahren nach Anspruch 1, wobei

   - der wenigstens eine Kennwert eine Gasmenge umfasst.

3. Verfahren nach Anspruch 2, wobei

   - die Gasmenge ermittelt wird durch Bestimmen eines Flächeninhalts (F), der von einer Kurve (AK) des zeitlichen Verlaufs der Widerstandstemperatur eingeschlossen wird.

4. Verfahren nach Anspruch 3, wobei

   - der Flächeninhalt (F) oberhalb eines vorbestimmten Temperaturmindestwerts ($T_{Gas}$) liegt.

5. Verfahren nach einem der Ansprüche 2 bis 4, wobei

   - die Gasmenge mit einem festgelegten Grenzwert verglichen wird,
   - eine Meldung ausgegeben wird, wenn die Gasmenge größer oder gleich dem festgelegten Grenzwert ist.

6. Verfahren nach einem der Ansprüche 1 bis 5, wobei

   - der wenigstens eine Kennwert eine Gaszusammensetzung umfasst.

7. Verfahren nach Anspruch 6, wobei

   - die Gaszusammensetzung ermittelt wird durch die Kurve (AK) des zeitlichen Verlaufs der Widerstandstemperatur und einer maximalen Widerstandstemperatur ($T_{max}$).

8. Verfahren nach einem der Ansprüche 1 bis 7, wobei

   - die Ermittlung der Widerstandstemperatur eine Messung des Laststromes umfasst.

9. Verfahren nach einem der Ansprüche 1 bis 8, wobei

   - die Ermittlung der Widerstandstemperatur eine Messung der Widerstandstemperatur umfasst.

10. Verfahren zur Zustandsbewertung eines mit Isoliermittel (IM) befüllten, elektrischen Betriebsmittels (EB), wobei das Betriebsmittel einen Laststufenschalter (S) umfasst und das Betriebsmittel (EB) und der Laststufenschalter (S) einen gemeinsamen Isoliermittelhaushalt (IM) aufweisen, und wobei

   - das Verfahren zur Zustandsbewertung ein Verfahren zur Überwachung von entstehenden Gasen (GG) gemäß einem der Ansprüche 1 bis 9 umfasst.

11. Verfahren nach Anspruch 10, wobei

- das Verfahren zur Zustandsbewertung außerdem eine Analyse des Isoliermittels (IM) umfasst,
- ein Ergebnis der Analyse abhängig von dem wenigstens einen Kennwert angepasst wird.

12. Laststufenschalter mit Überschaltwiderstand, wobei der Laststufenschalter (S) derart ausgestaltet ist, dass der Überschaltwiderstand (W) während dem Betrieb des Laststufenschalters mit einem Isoliermittelhaushalt (IM, IM') in Kontakt steht, wobei der Laststufenschalter eine Auswerteeinrichtung (AE) zur Überwachung entstehender Gase (GG, GG') in dem Isoliermittelhaushalt (IM, IM') aufweist, wobei die Auswerteeinrichtung (AE) dazu eingerichtet ist,

- einen Verlauf einer Widerstandstemperatur des Überschaltwiderstandes (W) während eines Belastungszeitraums zu ermitteln,
- aus dem zeitlichen Verlauf der Widerstandstemperatur wenigstens einen Kennwert zur Charakterisierung der entstehenden Gase (GG, GG') zu bestimmen.

13. Laststufenschalter nach Anspruch 12, wobei der Laststufenschalter (S) derart ausgelegt ist, dass er in einem elektrischen Betriebsmittel (EB) verbaut werden kann, wobei

- es sich bei dem Isoliermittelhaushalt (IM) um einen gemeinsamen Isoliermittelhaushalt von Betriebsmittel (EB) und Laststufenschalter (S) handelt.

14. Laststufenschalter nach Anspruch 13, wobei

- der Laststufenschalter (S) einen Temperatursensor (TS) zur Messung der Widerstandtemperatur umfasst.

**Claims**

1. A method for monitoring gases (GG, GG') produced in an insulating medium circuit (IM, IM'), wherein the insulating medium circuit (IM, IM') is in contact with a transition resistor (W) of an on-load tap-changer (S), and wherein

- a time profile of a resistor temperature of the transition resistor (W) during a loading time period is ascertained,
- at least one characteristic value for characterizing the gases (GG, GG') produced is determined from the time profile of the resistor temperature.

2. The method as claimed in claim 1, wherein

- the at least one characteristic value comprises a quantity of gas.

3. The method as claimed in claim 2, wherein

- the quantity of gas is ascertained by determining an area (F) which is enclosed by a curve (AK) of the time profile of the resistor temperature.

4. The method as claimed in claim 3, wherein

- the area (F) lies above a predetermined minimum temperature value ($T_{Gas}$).

5. The method as claimed in one of claims 2 to 4, wherein

- the quantity of gas is compared with a defined limit value,
- a notification is output when the quantity of gas is greater than or equal to the defined limit value.

6. The method as claimed in one of claims 1 to 5, wherein

- the at least one characteristic value comprises a gas composition.

7. The method as claimed in claim 6, wherein

- the gas composition is ascertained using the curve (AK) of the time profile of the resistor temperature and a

maximum resistor temperature ($T_{max}$).

8. The method as claimed in one of claims 1 to 7, wherein

    - ascertaining the resistor temperature comprises measuring the load current.

9. The method as claimed in one of claims 1 to 8, wherein

    - ascertaining the resistor temperature comprises measuring the resistor temperature.

10. A method for assessing the state of an item of electrical equipment (EB) filled with insulating medium (IM), wherein the item of equipment comprises an on-load tap-changer (S) and the item of equipment (EB) and the on-load tap-changer (S) have a common insulating medium circuit (IM), and wherein

    - the state assessment method comprises a method for monitoring gases (GG) produced as claimed in one of claims 1 to 9.

11. The method as claimed in claim 10, wherein

    - the state assessment method also comprises analysis of the insulating medium (IM),
    - a result of the analysis is adapted depending on the at least one characteristic value.

12. An on-load tap-changer having a transition resistor, wherein the on-load tap-changer (S) is configured in such a way that the transition resistor (W) is in contact with an insulating medium circuit (IM, IM') during operation of the on-load tap-changer, wherein the on-load tap-changer has an evaluation device (AE) for monitoring gases (GG, GG') produced in the insulating medium circuit (IM, IM'), wherein the evaluation device (AE) is designed

    - to ascertain a profile of a resistor temperature of the transition resistor (W) during a loading time period,
    - to determine at least one characteristic value for characterizing the gases (GG, GG') produced from the time profile of the resistor temperature.

13. The on-load tap-changer as claimed in claim 12, wherein the on-load tap-changer (S) is designed in such a way that it can be installed in an item of electrical equipment (EB), wherein

    - the insulating medium circuit (IM) is a common insulating medium circuit of the item of equipment (EB) and the on-load tap-changer (S).

14. The on-load tap-changer as claimed in claim 13, wherein

    - the on-load tap-changer (S) comprises a temperature sensor (TS) for measuring the resistor temperature.


**Revendications**

1. Procédé de surveillance de gaz produits (GG, GG') dans un bâti à agent isolant (IM, IM'), le bâti à agent isolant (IM, IM') étant en contact avec une résistance de commutation (W) d'un commutateur de gamme de charges (S), et

    - une variation dans le temps de la température de la résistance de commutation (W) étant déterminée pendant une période de charge,
    - au moins un paramètre de caractérisation des gaz produits (GG, GG') étant déterminé à partir de la variation dans le temps de la température de la résistance.

2. Procédé selon la revendication 1,

    - l'au moins un paramètre comprenant une quantité de gaz.

3. Procédé selon la revendication 2,

- la quantité de gaz étant déterminée par détermination d'une surface (F) qui est délimitée par une courbe (AK) de la variation dans le temps de la température de la résistance.

4. Procédé selon la revendication 3,

   - la surface (F) étant supérieure à une valeur de température minimale prédéterminée ($T_{Gas}$).

5. Procédé selon l'une des revendications 2 à 4,

   - la quantité de gaz étant comparée à une valeur limite spécifiée,
   - un message étant délivré si le volume de gaz est supérieur ou égal à la valeur limite spécifiée.

6. Procédé selon l'une des revendications 1 à 5,

   - l'au moins un paramètre comprenant une composition de gaz.

7. Procédé selon la revendication 6,

   - la composition de gaz étant déterminée par la courbe (AK) de la variation dans le temps de la température de la résistance et d'une température de résistance maximale ($T_{max}$).

8. Procédé selon l'une des revendications 1 à 7,

   - la détermination de la température de la résistance comprenant une mesure du courant de charge.

9. Procédé selon l'une des revendications 1 à 8,

   - la détermination de la température de résistance comprenant une mesure de la température de résistance.

10. Procédé d'évaluation de l'état d'un équipement électrique (EB) rempli d'un agent isolant (IM), l'équipement comprenant un commutateur de gamme de charges (S) et l'équipement (EB) et le commutateur de gamme de charges (S) comportant un bâti à agent isolant commun (IM), et

    - le procédé d'évaluation d'état comprenant un procédé de surveillance de gaz produits (GG) selon l'une des revendications 1 à 9.

11. Procédé selon la revendication 10,

    - le procédé d'évaluation d'état comprenant également une analyse de l'agent isolant (IM),
    - un résultat de l'analyse étant adapté en fonction de l'au moins un paramètre.

12. Commutateur de gamme de charges comprenant une résistance de commutation, le commutateur de gamme de charges (S) étant conçu de manière à ce que la résistance de commutation (W) soit en contact avec un bâti à agent isolant (IM, IM') pendant le fonctionnement du commutateur de gamme de charges, le commutateur de gamme de charges comportant un dispositif d'évaluation (AE) destiné à surveiller les gaz produits (GG, GG') dans le bâti à agent isolant (IM, IM'), le dispositif d'évaluation (AE) étant conçu pour

    - déterminer la variation de la température de la résistance de commutation (W) pendant une période de charge,
    - déterminer au moins un paramètre de caractérisation des gaz produits (GG, GG') à partir de la variation dans le temps de la température de résistance.

13. Commutateur de gamme de charges selon la revendication 12, le commutateur de gamme de charges (S) étant conçu de manière à pouvoir être installé dans un équipement électrique (EB),

    - le bâti à agent isolant (IM) étant un bâti à agent isolant commun de l'équipement (EB) et du commutateur de gamme de charges (S).

14. Commutateur de gamme de charges selon la revendication 13,

- le commutateur de gamme de charges (S) comprenant un capteur de température (TS) destiné à mesurer la température de la résistance.

**Fig. 1**

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3073248 A

**In der Beschreibung aufgeführte Nicht-Patentliteratur**

- Online Monitoring Data Cleaning of Transformer Considering Time Series Correlation. **LIN JUN et al.** 2018 IEEE/PES TRANSMISSION AND DISTRIBUTION CONFERENCE AND EXPOSITION (T&D). IEEE, 16. April 2018, 1-9

- **NAN YAO et al.** Transformer fault detection based on infrared power image. *ACTA TECHNICA,* 2017, vol. 62 (2A), 237-244